(19) Europäisches Patentamt
European Patent Office
Office européen des brevets

(11) Veröffentlichungsnummer : **0 374 096 B1**

(12) # EUROPÄISCHE PATENTSCHRIFT

(45) Veröffentlichungstag der Patentschrift : **02.12.92 Patentblatt 92/49**

(51) Int. Cl.[5] : **A61K 31/70**, // (A61K31/70, 31:52)

(21) Anmeldenummer : **89810920.2**

(22) Anmeldetag : **05.12.89**

(54) **2',3'-Dideoxypurinnucleosid/Purinnucleosid-Phosphorylase-Inhibitor Kombinationstherapie und Zusammensetzungen dafür.**

(30) Priorität : **14.12.88 US 284153**
**24.02.89 US 315354**

(43) Veröffentlichungstag der Anmeldung : **20.06.90 Patentblatt 90/25**

(45) Bekanntmachung des Hinweises auf die Patenterteilung : **02.12.92 Patentblatt 92/49**

(84) Benannte Vertragsstaaten : **AT BE CH DE FR GB IT LI LU NL SE**

(56) Entgegenhaltungen :
**EP-A- 0 149 775**
**EP-A- 0 216 510**

(73) Patentinhaber : **CIBA-GEIGY AG**
**Klybeckstrasse 141**
**CH-4002 Basel (CH)**

(72) Erfinder : **Erion, Mark D.**
**19 Hillside Avenue**
**Livingston New Jersey 07039 (US)**

EP 0 374 096 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Stoffgemisch bestehend aus einer antiretroviral wirksamen Menge eines 2′,3′-Dideoxypurinnucleosids und einer die Purinnucleosid-Phosphorylase inhibierenden Menge eines Purinnucleosid-Phosphorylase-Inhibitors, ein Erzeugnis enthaltend die einzelnen genannten Komponenten des Stoffgemisches, in An- oder Abwesenheit eines oder mehrerer pharmazeutisch annehmbarer Trägermaterialien, als Kombinationspräparat zur gleichzeitigen oder verschiedenzeitigen Anwendung innerhalb einer Zeitspanne, die klein genug ist, um zu gewährleisten, dass der Purinnucleosid-Phosphorylase-Inhibitor den Abbau des 2′,3′-Dideoxypurinnucleosids durch native Purinnucleosid-Phosphorylase verhindert oder wenigstens dessen Geschwindigkeit vermindert, zur Behandlung eines Zustandes, der auf Behandlung mit einem 2′, 3′-Dideoxypurinnucleotid anspricht; eine pharmazeutische Zusammensetzung und die Verwendung einer antiretroviral wirksamen Menge eines 2′,3′-Dideoxypurinnucleosids und einer die Purinnucleosid-Phosphorylase inhibierenden Menge eines Purinnucleosid-Phosphorylase-Inhibitors zur Herstellung von pharmazeutischen Präparaten zur Anwendung als Mittel gegen Retrovirusinfektionen.

Retrovirusinfektionen, unter Einbeziehung von HIV (Human Immunodeficiency Virus)Infektionen, können mit 2′,3′-Dideoxypurinnucleosiden behandelt werden. Auch Inhibitoren des Purinnucleosid-Stoffwechsels, insbesondere Purinnucleosid-Phosphorylase-Inhibitoren (PNP-Inhibitoren), sind als solche bekannt.

Erworbene Immunschwäche (acquired immunodeficiency syndrome oder AIDS) ist eine tödliche Krankheit. Sie ist durch ein Spektrum immunologischer Beschwerden gekennzeichnet, die sich nach einer Infektion mit HIV (human immunodeficiency virus) entwickeln [De Clercq, J.Med. Chem. 29, 1561 (1986)]. Verschiedene Strategien der Behandlung von AIDS werden gegenwärtig erforscht [Mitsuya & Broder, Nature 325, 773 (1987)]. Bis heute bedient sich die erfolgreichste chemotherapeutische Methode der 2′,3′-Dideoxynucleoside (ddN). Diese Verbindungen bilden das 5′-Triphosphat-2′,3′-dideoxy-Nucleotid (ddNT)-analoge. Dieses wiederum verhindert die HIV-DNA-Synthese und damit die Vermehrung des HIV, indem es entweder als Inhibitor oder als ein Substratanaloges für die reverse Transskriptase wirkt. Im letztgenannten Fall katalysiert die reverse Transskriptase die chemische Verknüpfung des ddNT mit dem 3′-Ende einer wachsenden DNA-Kette und beendet dadurch, wegen der Abwesenheit einer 3′-Hydroxygruppe, deren weitere Verlängerung.

Sowohl 3′-Azido-3′-deoxythymidin (AZT) als auch Dideoxycytidin (ddC) haben ihre Wirksamkeit am Menschen bei der Behandlung von HIV-Infektionen bewiesen, und von beiden 2′,3′-Dideoxypyrimidinnucleosid-Analogen wird berichtet, dass sie die Lebenszeit verlängern. Allerdings bringen beide auch einige ernste toxische Nebenwirkungen mit sich. AZT kann Myelosuppression und oft schwere Anämie verursachen, während ddC zu peripherer Neuropathie führen kann [Yarchoan et al., Lancet, 76 (1988)].

Im Vergleich zu AZT und ddC weisen 2′,3′-Dideoxypurinnucleoside, z.B. Dideoxyinosin (ddl) und Dideoxyadenosin (ddA) eher einen vielversprechenden therapeutischen Index in Zellkulturen auf [Mitsuya und Broder, Proc. Natl. Acad. Sci. 83, 1911 (1986)]. Andererseits sind ddl und ddA weniger wirksam beim Schutz von H9-Zellen vor HIV-Infektivität und von ATH8-Zellen vor den zytopathischen Wirkungen des HIV. Ein Grund für diese Abnahme der Wirksamkeit kann ihre metabolische Instabilität sein. Es wurde bereits gezeigt, dass Inkubation ganzer Zellen mit ddl oder ddA, welche im Purinring radiomarkiert sind, das mutmassliche Anti-HIV-Mittel 2′,3′-Dideoxyadenosin-5′-triphosphat (ddATP) neben wesentlichen Mengen von markiertem ATP und ADP ergibt. ddATP wurde vermutlich durch Umwandlung von ddl (ddA wird in vivo schnell in ddl umgewandelt) in ddlMP (2′,3′-Dideoxyinosin-Monophosphat) erzeugt, gefolgt vom Wiedereintritt in den Adenylat-Zyklus über Adenylsuccinatsynthetase [Ahluwalia et al., Biochem. Pharmac. 36, 3787 (1987)]. Man hat aber auch schon vermutet, dass markiertes ATP und ADP aus anfänglichem Abbau von ddl zu 2′,3′-Dideoxyribose- 1′-phosphat (ddRP) und Hypoxanthin (Hx) durch Purinnucleosid-Phosphorylase (PNP) entstehen. Das markierte Hypoxanthin wird danach über einen Stoffwechselweg, der im Englischen Purine-Salvage-Pathway genannt wird, in Adenylatnucleotide umgewandelt [Haertle et al., J. Biol. Chem. 263, 5870 (1988)]. Es ist bekannt, dass ddl ein PNP-Substrat ist. Hingegen ist ddl im Vergleich zu den natürlichen PNP-Substraten, Inosin und Guanosin, ein deutlich schlechteres [1000 mal weniger kcat/km; Stoeckler et al., Biochem. 19, 102 (1980)]. Der Umstand, dass dieses relativ schlechte PNP-Substrat in Zellkulturen in massgeblichem Umfang durch PNP abgebaut wird, legt nahe, dass entweder PNP in diesen Lymphozytzellinien in grossen Mengen vorliegt oder/und dass die ddl-Phosphorylierung zu ddlMP eine relativ langsame Reaktion ist.

Die Wirkung von PNP auf die Pharmakokinetik von ddl im vollständigen tierischen Organismus ist, im Gegensatz zu der in Zellkulturen, unbekannt. Die in vivo-Eliminierung von ddl könnte auf vielen verschiedenen Mechanismen beruhen, wie auf renaler Filtration, Oxidation, Konjugation, chemischer Hydrolyse usw. Wenn ein einziges Enzym den Abbau eines Wirkstoffes katalysieren würde und damit dessen in vivo-Halbwertszeit bestimmen würde, müsste der Wirkstoff ungehinderten Zugang zu dem Enzym haben, und die Umsatzrate müsste signifikant höher sein als die Rate aller anderen Mechanismen. Da aber ddl bekannterweise ein sehr schlechtes PNP-Substrat ist, lässt sich daraus schliessen, dass es unwahrscheinlich ist, dass die Geschwindigkeit der in vivo-Eliminierung von ddI von der PNP-Aktivität abhängt, und auch unwahrscheinlich, dass sie durch einen PNP-Inhibitor beeinflusst wird.

Eine beträchtliche Anzahl von Purinnucleosid-Phosphorylase (PNP) Inhibitoren ist bereits bekannt. Eine Anzahl dieser Verbindungen ist ausdrücklich offenbart in J. Stoecklers Kapitel in Developments in Cancer Chemotherapy, CRC Press, 1984, mit dem Titel "Purine Nucleoside Phosphorylase: A Target for Chemotherapy", Seiten 35 bis 60, und in Drugs of the Future 13, No. 7, 1988, "Purine Nucleoside Phosphorylase (PNP) Inhibitors: Potentially Selective Immunosuppressive Agents", Sircar und Gilbertsen, Seiten 653 bis 668; EP-A-0193454, EP-A-0178178, EP-A-0156559, EP-A-0145207 und EP-A-0260491.

Es wurde gefunden, dass PNP-Mangel oder -Inhibierung die durch T-Zellen vermittelte Immunität unterdrückt, wie in der vorstehend genannten Literatur berichtet und ferner in Cancer Research 46, Februar 1986 "Potentiation of 2′-Deoxyguanosine Cytotoxicity by a Novel Inhibition of Purine Nucleoside Phosphorylase, 8-amino-benzylguanine", Seiten 519 bis 523, Cancer Research 46, 1774 bis 1778, April 1986; Agents and Actions 21, 3/4 (1987), Seiten 253 bis 256; Agents and Actions 22, 3/4 (1987), Seiten 379 bis 384, Agents and Actions 21, 3/4 (1987), Seiten 272 bis 274; Immunology 1986, Band 59, Seiten 63 bis 67, und Clinical Experimental Immunology (1986), 66, Seiten 166 bis 172. Diese Zitate machen klar, dass ein PNP-Mangel in den Tod von T-Zellen mündet und die immunologische Unversehrtheit des Wirts beeinträchtigt. Zellen, denen PNP fehlt, sammeln toxische Mengen von Purinnucleotidtriphosphaten an, die den Zelltod verursachen. Tatsächlich scheinen T-Zellen diejenigen zu sein, die im erstaunlichsten Ausmass durch auf PNP-Mangel beruhende Vergiftungen beeinträchtigt werden. Daher scheint es auf der Hand zu liegen, dass eine Reduktion von PNP Aktivität in Patienten mit geschwächtem Immunsystem, wie AIDS-Patienten, kontraindiziert ist. Es erscheint daher ebenso kontraindiziert, PNP-Inhibitoren zu verabreichen, um eine Retrovirusinfektion, insbesondere eine HIV-Infektion, zu bekämpfen. Man könnte nämlich erwarten, dass PNP-Inhibitoren die Immunfunktionen bei solchen Infektionen zusätzlich beeinträchtigen.

Trotz der vorstehenden Ausführungen ist die Aufgabe und der Gegenstand der Erfindung darin zu sehen, Stoffgemische bestehend aus einer antiretroviral wirksamen Menge eines 2′,3′-Dideoxypurinnucleosids und einer die Purinnucleosid-Phosphorylase inhibierenden Menge eines Purinnucleosid-Phosphorylase-Inhibitors, Erzeugnisse enthaltend eine antiretroviral wirksame Menge eines 2′,3′-Dideoxypurinnucleosids und eine die Purinnucleosid-Phosphorylase inhibierende Menge eines Purinnucleosid-Phosphorylase-Inhibitors, in An- oder Abwesenheit eines oder mehrerer pharmazeutisch annehmbarer Trägermaterialien, als Kombinationspräparat zur gleichzeitigen oder verschiedenzeitigen Anwendung innerhalb einer Zeitspanne, die klein genug ist, um zu gewährleisten, dass der Purinnucleosid-Phosphorylase-Inhibitor den Abbau des 2′,3′-Dideoxypurinnucleosids durch native (körpereigene oder endogene) Purinnucleosid-Phosphorylase verhindert oder wenigstens dessen Geschwindigkeit vermindert, zur Behandlung eines Zustandes, der auf Behandlung mit einem 2′, 3′-Dideoxypurinnucleotid anspricht, und pharmazeutische Zusammensetzungen zur Behandlung eines Zustandes, der auf Behandlung mit einem 2′,3′-Dideoxypurinnucleosid anspricht, bestehend aus einer wirksamen Menge eines 2′,3′-Dideoxypurinnucleosids, einer die Purinnucleosid-Phosphorylase inhibierenden Menge eines Purinnucleosid-Phosphorylase-Inhibitors und einer oder mehreren pharmazeutisch annehmbaren Trägersubstanzen, bereitzustellen. Der zugrundeliegende Gedanke ist, dass ein Purinnucleosid-Phosphorylase-Inhibitor den metabolischen Abbau jenes 2′,3′-Dideoxypurinnucleosides inhibieren und dessen antiretrovirale Wirkung verstärken kann, obwohl die oben genannten Ausführungen das Gegenteil erwarten lassen.

Ein weiterer Gegenstand der Erfindung ist die Verwendung einer antiretroviral wirksamen Menge eines 2′,3′-Dideoxypurinnucleosids und einer die Purinnucleosid-Phosphorylase inhibierenden Menge eines Purinnucleosid-Phosphorylase-Inhibitors zur Herstellung von pharmazeutischen Präparaten zur Anwendung als Mittel gegen Retrovirusinfektionen, insbesondere gegen HIV-Infektionen.

Ein weiterer Gegenstand der Erfindung ist es, das oben genannte Stöffgemisch zur Anwendung in einem Verfahren zur therapeutischen Behandlung des menschlichen oder tierischen Körpers zur Verfügung zu stellen.

Überraschenderweise bewirken die oben genannten Stoffgemische und Erzeugnisse zur Anwendung zu den genannten Behandlungen bei dieser Anwendung die Verlangsamung des in-vivo-Stoffwechsels oder -Abbaus von 2′,3′-Dideoxypurinnucleosiden in Säugern, unter Einschluss des Menschen, die Erhöhung ihrer Wirkdauer, die Verstärkung ihrer therapeutischen Wirkung, die Verringerung der wirksamen Dosis, und die Minimierung der Nebenwirkungen, die bei einer Behandlung mit besagten 2′,3′-Dideoxypurinnucleosiden alleine beobachtet werden, und ferner die Verringerung der antiretroviral wirksamen Menge des 2′,3′-Dideoxypurinnucleosids auf eine solche, die bisher als subtherapeutisch gegolten hat.

Ein Zustand, welcher auf 2′,3′-Dideoxypurinnucleoside anspricht, ist vor allem eine Retrovirusinfektion, insbesondere bei Säugern, unter Einschluss des Menschen, vorzugsweise Infektion durch HIV (AIDS-Virus).

Ein 2′,3′-Dideoxypurinnucleosid (ddPN) ist beispielsweise eine Verbindung der allgemeinen Formel I,

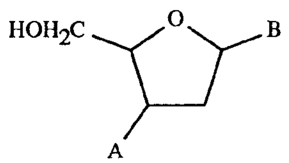

(I)

worin A ein Wasserstoffatom, eine Azidgruppe oder ein Halogenatom, vorzugsweise ein Wasserstoffatom, ist, B den Rest einer Purinbase bedeutet, die über ihre 9-Stellung an den Dideoxy-Zuckerrest gebunden ist, und die Hydroxygruppe entweder frei oder als ein metabolisierbarer Ester derivatisiert ist, oder ein pharmazeutisch annehmbares Salz davon. Ein Ester einer Verbindung der allgemeinen Formel I ist eine Verbindung der allgemeinen Formel I, worin die Hydroxygruppe der Hydroxymethlygruppe mit eine Säure verestert ist, und ist a) ein Carbonsäureester einer Säure der Formel $R_1$-COOH, worin $R_1$ unverzweigtes und verzweigtes Alkyl, Alkoxyalkyl, Arylalkyl, Aryloxyalkyl oder Aryl bedeutet, worin Aryl für Phenyl steht oder für Naphthyl, welche unsubstituiert oder durch Halogen, Alkyl oder Alkoxy substituiert sind, z. B. ein Acetat, 3-Methylbutyrat, Octanoat, Palmitat, 3-Chlorbenzoat, 4-Methylbenzoat, Hydrogensuccinat oder Pivalat, b) ein Sulfonatester einer Sulfonsäure der Formel $R_2$-$SO_3H$, worin $R_2$ Alkyl oder Arylalkyl bedeutet, worin Aryl wie vorstehend definiert ist, z. B. ein Mesylat, oder c) ein Phosphatester der Phosphorsäure der Formel

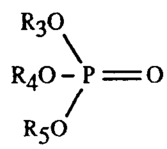

worin mit der Massgabe, dass wenigstens einer der Reste $R_3$ bis $R_5$ von Wasserstoff verschieden ist, $R_3$ bis $R_5$ unabhängig voneinander jeweils ein Wasserstoffatom oder einen Rest mit einer der Bedeutungen, die vorstehend für $R_1$ angegeben sind, symbolisieren, z. B. ein Monophosphat, Diphosphat oder Triphosphat. Im Zusammenhang mit den vorstehenden Definitionen weisen Alkyl und Alkoxy jeweils 1 bis 18, vorzugsweise 1 bis 7 und insbesondere 1 bis 4 Kohlenstoffatome auf. Halogen bedeutet vorzugsweise Fluor oder Chlor. Der Rest einer Purinbase B steht vorzugsweise für Adenin-9-yl, Guanin-9-yl, Hypoxanthin-9-yl, Thiohypoxanthin-9-yl, Thioguanin-9-yl oder 2-Aminoadenin-9-yl. Bevorzugt als ddPN ist eine Verbindung der allgemeinen Formel I, worin A ein Wasserstoffatom bedeutet, B für Thioguanin-9-yl oder 2-Aminoadenin-9-yl steht und die Hydroxygruppe frei ist, also entweder 2′,3′-Dideoxythioguanosin oder 2′,3′-Dideoxy-2-aminoadenosin, oder ein pharmazeutisch annehmbares Salz davon. Bevorzugt als ddPN ist auch eine Verbindung der allgemeinen Formel I, worin A ein Wasserstoffatom bedeutet, B für Adenin-9-yl, Guanin-9-yl, Hypoxanthin-9-yl oder Thiohypoxanthin-9-yl steht, und die Hydroxygruppe frei ist, also entweder 2′,3′-Dideoxyadenosin, 2′,3′-Dideoxyguanosin, 2′,3′-Dideoxyinosin oder 2′,3′-Dideoxythioinosin, oder ein pharmazeutisch annehmbares Salz davon.

Am stärksten bevorzugt ist die entsprechende Verbindung, worin A ein Wasserstoffatom bedeutet, B für Adenin-9-yl steht und die Hydroxygruppe frei ist, d.h. 2′,3′-Dideoxyadenosin, und ein pharmazeutisch annehmbares Salz davon. Ebenso bevorzugt ist die entsprechende Verbindung, worin A ein Wasserstoffatom bedeuted, B für Hypoxanthin-9-yl steht und die Hydroxygruppe frei ist, d. h. 2′,3′-Dideoxyadenosin, oder ein pharmazeutisch annehmbares Salz davon.

Die vorstehend genannten Verbindungen sind bekannt oder können auf an sich bekannte Weise hergestellt werden, beispielsweise wie in der EP-A-0206 497, publiziert am 30.12.1986, beschrieben.

Spezielle Beispiele von pharmazeutisch annehmbaren Salzen einer Verbindung der allgemeinen Formel I sind pharmazeutisch annehmbare Alkalimetallsalze, vorzugsweise die Mononatriumsalze, von 5′-Estern mit entsprechenden Resten, z. B. einem Mono-, Di-oder Triphosphat, oder z. B. ein entsprechendes Dinatriummonophosphat.

Ein Purinnucleosid-Phosphorylase (PNP)-Inhibitor lässt sich unter einer Vielzahl von Verbindungen finden, von denen viele bereits bekannt sind. Eine Anzahl dieser Verbindungen ist ausdrücklich offenbart in J. Stoeckers Kapitel in Developments in Cancer Chemotherapy, CRC Press, 1984, mit dem Titel "Purine Nucleoside Phosphorylase: A Target for Chemotherapy", Seiten 35 bis 60, und in Drugs of the Future 13, No. 7, 1988, "Purine Nucleoside Phosphorylase (PNP) Inhibitors: Potentially Selective Immunosuppressive Agents", Sircar und Gilbertsen, Seiten 653 bis 668; verwiesen wird auch auf die US Patentanmeldung 704,991, eingereicht am 25. Februar 1985, die der EP-A-0193454 entspricht, auf die US Patentanmeldung 660,152, eingereicht am 12. Oktober 1984 und die US Patentanmeldung 767,202, eingereicht am 22. August 1985, die der EP-A-0178178 entsprechen, auf die US Patentanmeldung 593,063, eingereicht am 26. März 1984 und die US Patentanmeldung 698,905, eingereicht am 11. Februar 1985, die der EP-A-0156559 entsprechen, auf die US Patentanmeldung 546,297, eingereicht am 31. Oktober 1983 und die US Patentanmeldung 657,211, eingereicht am 19. Juni 1985, die der EP-A-0145207 entsprechen und auf die US Patentanmeldung 900,486, eingereicht am 26. Juni 1986 und die US Patentanmeldung 59,419, eingereicht am 18. Juni 1987, die der EP-A-0260491 entsprechen; wobei ein 9-substituiertes Guaninderivat ein Beispiel ist, z.B. wie in der EP-A-0178178 (US-A-4,722,606) offenbart, in der EP-A-0156559, Agents and Actions, 21, 3/4 (1987), Seiten 253-256 oder in Drugs of the Future 13, 653 (1988), oder ein 9-substituiertes-9-Deazaguaninderivat gemäss EP-A-0260491, z.B. eine Verbindung der folgenden allgemeinen Formel,

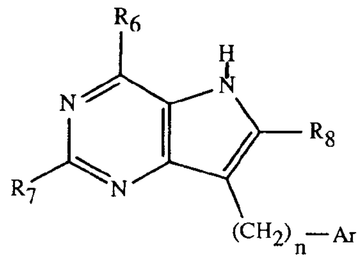

worin $R_6$ OH oder SH ist, $R_7$ für ein Wasserstoffatom oder $NH_2$ steht, $R_8$ ein Wasserstoffatom oder $NH_2$ ist, n für eine ganze Zahl von null bis vier steht und Ar entweder (i) unsubstituiertes oder durch Halogen, Alkyl mit ein bis vier Kohlenstoffatomen, Hydroxy, Alkoxy mit ein bis vier Kohlenstoffatomen, oder Trifluormethyl-substituiertes Phenyl ist oder (ii) 2- oder 3-Thienyl oder (iii) 2- oder 3-Furanyl ist, oder ein pharmazeutisch annehmbares Salz davon, oder insbesondere eine derartige Verbindung, in der $R_6$ OH ist und n für eins steht, insbesondere ein 9-Benzyl-, 9-(2-Thienylmethyl)- oder 9-(3-Thienylmethyl)-analoges von 8-Aminoguanosin oder 8-Aminoguanin, oder ein 9-Benzyl-, 9-(2-Thienylmethyl)- oder 9-(3-Thienylmethyl)-analoges von 8-Amino-9-deazaguanin, oder 8-Amino-9-deazaguanin, 8-Aminoguanosin oder 8-Aminoguanin selbst.

Erfindungsgemäss kann jede PNP-inhibierende Verbindung verwendet werden. PNP-Inhibitoren mit einem $IC_{50}$-Wert von weniger als 5 μm für die Hemmung von PNP sind bevorzugt.

Ein bevorzugter PNP-Inhibitor ist entweder (a) Formycin B, oder (b) 8-Aminoguanin, 8-Aminoguanosin, 1-β-D-Ribofuranosyl-1H-1,2,4-triazol-3-carboxamidin, 8-Amino-9-(1,3-dihydroxy-2-propoxymethyl)guanin, 9-Deazaguanosin, 9-Deazainosin, 5′-Deoxy-5′-chlor-9-deazainosin, 5′-Deoxy-5′-iod-9-deazainosin oder 8-Amino-9-deaza-9-(3-thienylmethyl)guanin, oder (c) 8-Amino-9-benzylguanin, 8-Amino-9-(2-thienylmethyl) guanin oder 8-Amino-9-(3-thienylmethyl)guanin. Von diesen Verbindungen ist ein PNP-Inhibitor der Gruppe (b) oder (c) stärker bevorzugt. Am meisten bevorzugt ist ein PNP-Inhibitor der Gruppe (c).

Die Verstärkung der antiretroviralen Wirksamkeit eines 2′,3′-Dideoxypurinnucleosids, beispielsweise eines solchen der allgemeinen Formel I, durch einen Purinnucleosid-Phosphorylase-Inhibitor (PNP-Inhibitor) kann beispielsweise in Zellkulturen (z.B. H9 Zellen, ATH8 Zellen) bestimmt werden, die einem Retrovirus (z.B. HIV) ausgesetzt werden. Die Methodik dafür ist an sich bereits beschrieben, z.B. in Proc. Nat. Acad. Sci. USA, 83, 1911 (1986). Die Verstärkung kann auch in vivo bestimmt werden (z.B. in Ratten), indem der Anstieg des Plasmalevels des Dideoxypurinnucleosids gemessen wird, der durch vorgängige oder gleichzeitige Verabreichung eines bestimmten PNP-Inhibitors erreicht wird. Auch diese Bestimmung und die Methoden dafür sind bereits Stand der Technik.

Die gezielte Inhibierung von verfügbarem PNP in einem durch einen Retrovirus befallenen Wirt, insbesondere in einem durch HIV befallenen Wirt, kann daher massgeschneidert eingesetzt werden, so dass 2′,3′-Dideoxypurinnucleoside in infizierten Zellen einen ausreichenden antiretroviralen Effekt aufweisen, ohne nicht infizierte Zellen massgeblich zu beeinträchtigen.

Die erfindungsgemäss verabreichbaren Verbindungen können zur Anwendung zur Therapie auf beliebige geeignete Weise verabreicht werden, unter anderem oral, rektal, nasal, topisch, (unter Einschluss von buccal und sublingual), vaginal, parenteral (unter Einschluss von subkutan, intramuskulär, intravenös und intradermal) und transdermal. Es liegt klar auf der Hand, dass die im Einzelfall bevorzugte Art der Verabreichung vom Zustand und Alter des Patienten abhängt sowie von der Art der Infektion und dem gewählten aktiven Wirkstoff.

Im allgemeinen bewegt sich eine geeignete Dosis von ddPN im Bereich von etwa 0,01 bis 20 mg pro Kilogramm Körpergewicht des Empfängers und Tag, vorzugsweise im Bereich von etwa 0,05 bis 10 mg pro Kilogramm Körpergewicht und Tag, und besonders bevorzugt im Bereich von etwa 0,1 bis 5 mg pro Kilogramm Körpergewicht und Tag. Die gewünschte Dosis wird vorzugsweise in Form von zwei, drei, vier, fünf, sechs oder mehr Teildosen bereitgestellt, die in geeigneten Intervallen während eines Tages verabreicht werden. Diese Teildosen können in Einheitsdosisformen verabreicht werden, beispielsweise enthaltend etwa 0,1 bis 200 mg,

vorzugsweise etwa 0,5 bis 100 mg und besonders bevorzugt etwa 1,0 bis 50 mg aktiver Wirksubstanz pro Einheitsdosisform.

Idealerweise sollte der ddPN-Bestandteil in einer Weise verabreicht werden, die dazu führt, dass Spitzenplasmakonzentrationen der aktiven Verbindung von etwa 1 bis 75 Mikromol, vorzugsweise etwa 2 bis 50 Mikromol und besonders bevorzugt etwa 3 bis etwa 30 Mikromol erreicht werden.

Typischerweise wird der PNP-Inhibitor in einer Menge von etwa 0,5 bis 150 mg/kg/Tag verabreicht, die in so viele Dosen unterteilt werden kann wie üblich und angemessen. In der vorteilhaftesten Ausführungsform inhibiert die verabreichte PNP-Inhibitormenge nicht mehr als etwa 98 % der verfügbaren PNP-Aktivität. Der PNP-Inhibitor wird in einer Dosis verabreicht, die vorzugsweise etwa 50-98 %, besonders bevorzugt etwa 65-95 % und vorteilhafterweise 75 bis 95 % der verfügbaren PNP-Aktivität inhibiert.

Es ist möglich, dass jede aktive Wirksubstanz allein vorliegt. Es ist aber bevorzugt, dass sie als pharmazeutische Formulierung vorliegt. Der PNP-Inhibitor kann alternativ einfach als zweiter Wirkstoff in dieselbe Formulierung zusammen mit dem 2′,3′-Dideoxypurinnucleosid eingebracht werden. Die erfindungsgemäßen Formulierungen beinhalten wenigstens eine aktive Wirksubstanz, wie oben definiert, zusammen mit einer oder mehreren pharmazeutisch annehmbaren Trägersubstanzen. Jeder Träger muss annehmbar in dem Sinne sein, dass er mit den anderen Bestandteilen der Formulierung verträglich und für den Patienten nicht schädlich ist. Formulierungen sind z. B. solche, die für orale, rektale, nasale, topische (unter Einschluss von bukkaler und sublingualer), vaginale, parenterale (unter Einschluss von subkutaner, intramuskulärer, intravenöser und intradermaler) oder transdermale Verabreichung geeignet sind. Die Formulierung kann üblicherweise in Dosiseinheitsform angeboten werden. Ferner kann sie nach einem beliebigen in der Pharmazie bekannten Verfahren hergestellt werden. Solche Verfahren umfassen den Schritt, bei dem der aktive Wirkstoff mit dem Träger zusammengebracht wird, welcher einen oder mehrere zusätzliche Bestandteile umfasst. Im allgemeinen werden die Formulierungen hergestellt, indem der aktive Wirkstoff einheitlich und innig mit flüssigen Trägern oder fein verteilten festen Trägem oder beidem vermischt wird. Danach wird das Produkt, falls erforderlich, in eine bestimmte Form gebracht.

Bevorzugte erfindungsgemässe pharmazeutische Zusammensetzungen (d. h. entsprechende pharmazeutische Formulierungen) zur Behandlung eines Zustandes, der auf Behandlung mit einem 2′,3′-Dideoxypurinnucleosid anspricht, bestehend aus einer wirksamen Menge eines 2′, 3′-Dideoxypurinnucleosids, einer die Purinnucleosid-Phosphorylase inhibierenden Menge eines Purinnucleosid-Phosphorylase-Inhibitors und einer oder mehreren pharmazeutisch annehmbaren Trägersubstanzen, sind solche, worin das 2′,3′-Dideoxypurinnucleosid 2′,3′-Dideoxyadenosin, 2′,3′-Dideoxyguanosin, 2′,3′-Dideoxyinosin, 2′,3′-Dideoxythioinosin, 2′,3′-Dideoxythioguanosin oder 2′,3′-Dideoxy-2-aminoadenosin ist, insbesondere 2′,3′-Dideoxyinosin, oder solche, worin der Purinnucleosid-Phosphorylase-Inhibitor entweder (a) Formycin B, oder (b) 8-Aminoguanin, 8-Aminoguanosin, 1-β-D-Ribofuranosyl-1H-1,2,4-triazol-3-carboxamidin, 8-Amino-9-(1,3-dihydroxy-2-propoxymethyl)guanin, 9-Deazaguanosin, 9-Deazainosin, 5′-Deoxy-5′-chlor-9-deazainosin, 5′-Deoxy-5′-od-9-deazainosin oder 8-Amino-9-deaza-9-(3-thienylmethyl)-guanin oder (c) 8-Amino-9-benzylguanin, 8-Amino-9-(2-thienylmethyl)guanin oder 8-Amino-9-(3-thienylmethyl)guanin ist. Besonders bevorzugt sind pharmazeutische Zusammensetzungen, worin das 2′,3′-Dideoxypurinnucleosid 2′,3′-Dideoxyadenosin 2′,3′-Dideoxyguanosin, 2′,3′-Dideoxyinosin, 2′,3′-Dideoxythioinosin, 2′,3′-Dideoxythioguanosin oder 2′,3′-Dideoxy-2-aminoadenosin und der Purinnucleosid-Phosphorylase-Inhibitor entweder (a) Formycin B oder (b) 8-Aminoguanin, 8-Aminoguanosin, 1-β-D-Ribofuranosyl- 1H- ,2,4-triazol-3-carboxamidin, 8-Amino-9-(1,3-dihydroxy-2-propoxymethyl) guanin, 9-Deazaguanosin, 9-Deazainosin, 5′-Deoxy-5′-chlor-9-deazainosin, 5′-Deoxy-5′-iod-9-deazainosin oder 8-Amino-9-deaza-9-(3-thienylmethyl)guanin oder (c) 8-Amino-9-benzylguanin, 8-Amino-9-(2-thienylmethyl)guanin oder 8-Amino-9-(3-thienylmethyl)guanin ist.

Erfindungsgemässe Formulierungen, die für orale Verabreichung geeignet sind, können als diskrete Einheiten zur Verfügung gestellt werden, wie als Kapseln, verschweisste Umschläge (cachets) oder Tabletten, von denen jede einzelne eine bestimmte Menge der aktiven Wirksubstanz enthält, als Puder oder Granulat, als Lösung oder Suspension in einer wässrigen oder nicht-wässrigen Flüssigkeit, oder als flüssige Oel-in-Wasser-Emulsion oder als flüssige Wasser-in-Oel-Emulsion. Der aktive Wirkstoff kann auch als Bolus, Elektuar oder Paste verabreicht werden.

Eine Tablette kann durch Verpressen oder Giessen hergestellt werden, wahlweise mit einem oder mehreren zusätzlichen Bestandteilen. Verpresste Tabletten können hergestellt werden, indem der aktive Wirkstoff in freifliessender Form, wie als Puder oder Granulat, wahlweise vermischt mit einem Bindemittel (z.B. Povidon, Gelatine, Hydroxypropylmethylcellulose), einem Gleitmittel, inertem Verdünnungsmittel, einem Konservierungsstoff, einem Zersetzungsmittel (z.B. Natriumstärkeglycollat, quervernetztes Povidon, quervernetzte Natriumcarboxymethylcellulose), einem oberflächenaktiven oder dispergierenden Mittel, mit einer geeigneten Maschine verpresst wird. Gegossene Tabletten können hergestellt werden, indem ein Gemisch der pulverisierten Verbindung, angefeuchtet mit einem inerten flüssigen Verdünnungsmittel, mit einer geeigneten Maschine

gegossen wird. Die Tabletten können wahlweise umhüllt oder eingekerbt sein. Ferner können sie so zusammengesetzt sein, dass der aktive Wirkstoff langsam oder kontrolliert freigegeben wird. Dafür wird beispielsweise Hydroxypropylmethylcellulose in unterschiedlichen Mengenverhältnissen verwendet, um das gewünschte Freisetzungsprofil zu erreichen. Tabletten können unter Umständen mit einem Überzug versehen sein, um in anderen Teilen des Verdauungstraktes als im Magen Freisetzung zu bewirken. Dies ist besonders vorteilhaft im Zusammenhang mit Verbidungen der allgemeinen Formel I, da solche Verbindungen gegenüber Säurehydrolyse empfindlich sind.

Formulierungen, die für die topische Verabreichung im Mund geeignet sind, umfassen Pastillen, die den aktiven Wirkstoff in einer geschmacksverbessernden Grundlage enthalten, üblicherweise mit Sucrose und Akazia oder Traganth, Pastillen, enthaltend den aktiven Wirkstoff in einer inerten Grundlage wie Gelatine und Glycerin, oder Sucrose und Akazia, und Mundspülungen, enthaltend die aktive Wirksubstanz in einem geeigneten flüssigen Träger.

Formulierungen, die für rektale Verabreichung geeignet sind, können als Suppositorien mit einer geeigneten Grundlage zur Verfügung gestellt werden, umfassend zum Beispiel Kokosbutter oder ein Salicylat.

Formulierungen, die für vaginale Verabreichung geeignet sind, können als Pessare, Tampons, Creme, Gele, Pasten, Schäume oder Sprays zur Verfügung gestellt werden, die zusätzlich zum ddPN-Bestandteil Träger enthalten, von denen bereits bekannt ist, dass sie für derartige Zwecke geeignet sind.

Formulierungen, die für parenterale Verabreichung geeignet sind, umfassen wässrige und nicht-wässrige isotonische sterile Injektionslösungen, die Antioxidantien, Puffer, Bakteriostatika und gelöste Stoffe enthalten können, die die Formulierung auf Isotonanz mit dem Blut des Empfängers einstellen, ferner wässrige und nicht-wässrige sterile Suspensionen, die zusätzlich Suspendierungsmittel und Verdicker enthalten können. Die Formulierungen können in verschlossenen Einzeldosisbehältern oder Multidosisbehaltern angeboten werden, beispielsweise in Ampullen oder Vials, und können gefriergetrocknet (lyophilisiert) aufbewahrt werden, so dass man nur einen sterilen flüssigen Träger, beispielsweise Wasser für Injektionen, unmittelbar vor der Anwendung hinzugeben muss. In situ (unmittelbar vor Applikation) herzustellende Injektionslösungen und -suspensionen können aus sterilen Pudern, Granulaten und Tabletten der vorstehend beschriebenen Art hergestellt werden.

Bevorzugte Einheitsdosisformulierungen sind solche, die eine Tagesdosis oder -einheit, eine Tagesteildosis oder einen geeigneten Teil davon von einem ddPN-Bestandteil und/oder einem PNP-Inhibitor enthalten.

Die beiden aktiven Bestandteile (ddPN und PNP-Inhibitor) können zur gleichzeitigen oder zur verschiedenzeitigen Anwendung zur Behandlung, wie oben definiert, auf die gleiche oder auf verschiedene Weisen, in derselben oder in unterschiedlichen Formulierungen, gelangen, unter der Voraussetzung, dass eine wirksame PNP-Inhibierung besteht, wenn das 2′,3′-Dideoxypurinnucleosid anwesend ist und wirkt. Die Zeitspanne, in der die beiden aktiven Wirkstoffe zur Anwendung gelangen, hängt von den Menge des verfügbaren PNP ab und von der Geschwindigkeit, mit der der PNP-Inhibitor selbst abgebaut wird. Aus diesen Gründen besteht die bevorzugte Dosierung in der Gabe von unterteilten Tagesdosen zwei bis vier Mal am Tage, wobei es besonders. bevorzugt ist, beide Wirkstoffe gleichzeitig zur Anwendung zur Behandlung gelangen zu lassen.

Die nachfolgenden Beispiele sollen dem besseren Verständnis der Erfindung dienen.

Beispiel 1:

(a) Herstellung von 10000 Kapseln :

| | |
|---|---|
| 2',3'-Dideoxyadenosin | 60 g |
| 8-Amino-9-(2-thienylmethyl)guanin | 1400 g |

Die beiden Komponenten werden miteinander vermischt und in Kapseln gefüllt, die beispielsweise für eine Verabreichung von 2 Kapseln alle 8 Stunden geeignet sind, d.h. dreimal täglich für einen Erwachsenen.

Auf ähnliche Weise werden Formulierungen von anderen 2′,3′-Dideoxypurinnucleosiden, z.B. von Dideoxyinosin, in Kombination mit einem geeigneten PNP-Inhibitor hergestellt.

(b) für 10000 Kapseln:

| | |
|---|---|
| 2',3'-Dideoxyinosin | 50 g |
| 8-Amino-9-deaza-9-(3-thienylmethyl)-guanin (Verbindung 9 der EP-A-0 260 491) | 2000 g |

(c) für 10000 Kapseln:

| | |
|---|---|
| 2',3'-Dideoxyinosin | 50 g |
| 8-Amino-9-(2-thienylmethyl)guanin | 1500 g |

Beispiel 2:

Die Erhöhung der antiretroviralen Aktivität von Dideoxynucleosiden durch einen PNP-Inhibitor kann in Zellkulturen beispielsweise wie folgt bestimmt werden:

H9 Zellen [Popovic et al., Science 224, 497 (1984)] oder ATH8 Zellen [Mitsuye und Broder, Proc. Natl. Acad. Sci. USA, 83, 1911 (1986)] werden mit einem bestimmten Dideoxypurinnucleosid (ddPN) entweder allein oder in Kombination mit einer für Purinnucleosid-Phosphorylase-Inhibierung wirksamen Menge eine PNP-Inhibitors behandelt. Die Zellen werden dann dem HIV ausgesetzt. Nach Inkubation während einiger Tage wird gemäss dem experimentellen Protokoll von Mitsuye und Broder, Proc. Natl. Acad. Sci. USA 83, 1911 (1986) die Anzahl von lebensfähigen Zellen bestimmt. Der Umstand, dass in der Zellkultur, die mit der Kombination von ddPN und PNP-Inhibitor behandelt wurde, eine grössere Anzahl lebensfähiger Zellen gefunden wird, verglichen mit der Kultur, die mit ddPN allein behandelt wurde, weist auf eine Verstärkung der Wirkung von ddPN durch den PNP-Inhibitor beim Schutz solcher Zellen vor der cytopathischen Wirkung des HIV hin.

Beispiel 3:

Die Wirkung eines Purinnucleosid-Phosphorylase (PNP) Inhibitors auf die Plasmakonzentration eines Dideoxypurinnucleosids (ddPN) kann wie folgt bestimmt werden:

Einer Gruppe von Lewis-Ratten wird entweder i.v., p.o. oder i.p. eine nicht-toxische Dosis des bestimmten antiretroviralen ddPN verabreicht. Einer anderen Gruppe wird die gleiche Menge des bestimmten ddPN verabreicht, nachdem man ihr zuvor entweder i.v., p.o. oder i.p. eine Purinnucleosid-Phosphorylase inhibierende Menge eines PNP-Inhibitors verabreicht hat. Blutproben werden in verschiedenen Abständen entnommen und mit HPLC auf die Plasmakonzentration des ddPN in beiden Gruppen untersucht.

Eine Erhöhung und/oder eine verlängerte Aufrechterhaltung des Plasmalevels von ddPN in der Gruppe von Ratten, die sowohl mit ddPN als auch mit PNP-Inhibitor behandelt wurden, weist darauf hin, dass der Stoffwechselabbau von ddPN inhibiert wird. Damit wird gleichzeitig gezeigt, dass die therapeutische Wirksamkeit des bestimmten ddPN durch den ebenfalls verabreichten PNP-Inhibitor erhöht wird.

Beispielsweise werden in Ratten, denen i.v. 20 mg/kg 2′,3′-Dideoxyinosin und i.v. etwa 5 bis 10 mg/kg 8-Amino-9-(2-thienylmethyl)guanin verabreicht wurden, die Plasmalevel von 2′,3′-Dideoxyinosin erhöht und länger aufrechterhalten im Vergleich zu Ratten, denen allein die gleiche Dosis von 2′,3′-Dideoxyinosin verabreicht wurde.

## Patentansprüche

1. Stoffgemisch bestehend aus einer antiretroviral wirksamen Menge eines 2′,3′-Dideoxypurinnucleosids und einer die Purinnucleosid-Phosphorylase inhibierenden Menge eines Purinnucleosid-Phosphorylase-Inhibitors.

2. Stoffgemisch gemäss Anspruch 1, worin das 2′,3′-Dideoxypurinnucleosid eine Verbindung der allgemeinen Formel I ist,

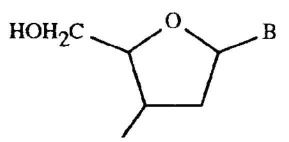

(I)

worin A ein Wasserstoffatom, eine Azidgruppe oder ein Halogenatom bedeutet, B den Rest einer Purinbase bedeutet, die über ihre 9-Stellung gebunden ist, und die Hydroxygruppe entweder frei oder als ein metabolisierbarer Ester derivatisiert ist, oder ein pharmazeutisch annehmbares Salz davon.

3. Stoffgemisch gemäss Anspruch 2, worin die Verbindung der allgemeinen Formel I entweder 2′,3′-Dideoxythioguanosin oder 2′,3′-Dideoxy-2-aminoadenosin ist.

4. Stoffgemisch gemäss Anspruch 2, worin die Verbindung der allgemeinen Formel I 2′,3′-Dideoxyadenosin, 2′,3′-Dideoxyguanosin, 2′,3′-Dideoxyinosin oder 2′,3′-Dideoxythioinosin ist.

5. Stoffgemisch gemäss Anspruch 2, worin die Verbindung der allgemeinen Formel I 2′,3′-Dideoxyadenosin ist.

6. Stoffgemisch gemäss Anspruch 2, worin die Verbindung der allgemeinen Formel I 2′,3′-Dideoxyinosin ist.

7. Stoffgemisch gemäss einem der Ansprüche 1 bis 6, worin der Purinnucleosid-Phosphorylase-Inhibitor entweder (a) Formycin B oder (b) 8-Aminoguanin, 8-Aminoguanosin, 1-β-D-Ribofuranosyl-1H- 1,2,4-triazol-3-carboxamidin, 8-Amino-9-(1,3-dihydroxy-2-propoxymethyl)guanin, 9-Deazaguanosin, 9-Deazainosin, 5′-Deoxy-5′-chlor-9-deazainosin, 5′-Deoxy-5′-iod-9-deazainosin oder 8-Amino-9-deaza-9-(3-thienylmethyl)guanin oder (c) 8-Amino-9-benzylguanin, 8-Amino-9-(2-thienylmethyl)guanin oder 8-Amino-9-(3-thienylmethyl)guanin ist.

8. Stoffgemisch gemäss Anspruch 7, worin der Purinnucleosid-Phosphorylase-Inhibitor entweder eine Verbindung der Gruppe (b) oder der Gruppe (c) ist.

9. Stoffgemisch gemäss Anspruch 7, worin der Purinnucleosid-Phosphorylase-Inhibitor eine Verbindunge der Gruppe (c) ist.

10. Stoffgemisch gemäss Anspruch 1, worin die antiretroviral wirksame Menge des 2′,3′-Dideoxypurinnucleosids 0,01 bis 20 mg und die die Purinnucleosid-Phosphorylase inhibierende Menge des Purinnucleosid-Inhibitors 0,5 bis 150 mg beträgt.

11. Stoffgemisch, bestehend aus einer antiretroviral wirksamen Menge eines 2′,3′-Dideoxypurinnucleosides und einer die Purinnucleosid-Phosphorylase inhibierenden Menge eines Purinnucleosid-Phosphorylase-Inhibitors, zur therapeutischen Behandlung des menschlichen oder tierischen Körpers.

12. Erzeugnis, enthaltend eine antiretroviral wirksame Menge eines 2′,3′-Dideoxypurinnucleosids und eine die Purinnucleosid-Phosphorylase inhibierende Menge eines Purinnucleosid-Phosphorylase-Inhibitors, in An- oder Abwesenheit eines oder mehrerer pharmazeutisch annehmbarer Trägermaterialien, als Kombinationspräparat zur gleichzeitigen oder verschiedenzeitigen Anwendung innerhalb einer Zeitspanne, die klein genug ist, um zu gewährleisten, dass der Purinnucleosid-Phosphorylase-Inhibitor den Abbau des 2′,3′-Dideoxypurinnucleosids durch native Purinnucleosid-Phosphorylase verhindert oder wenigstens dessen Geschwindigkeit vermindert, zur Behandlung eines Zustandes, der auf Behandlung mit einem 2′, 3′-Dideoxypurinnucleotid anspricht.

13. Pharmazeutische Zusammensetzung zur Behandlung eines Zustandes, der auf Behandlung mit einem 2′,3′-Dideoxypurinnucleosid anspricht, bestehend aus einer wirksamen Menge eines 2′,3′-Dideoxypurinnucleosids, einer die Purinnucleosid-Phosphorylase inhibierenden Menge eines Purinnucleosid-Phosphorylase-Inhibitors und einer oder mehreren pharmazeutisch annehmbaren Trägersubstanzen.

14. Pharmazeutische Zusammensetzung gemäss Anspruch 13, worin das 2′,3′-Dideoxypurinnucleosid 2′,3′-Dideoxyadenosin, 2′,3′-Dideoxyguanosin, 2′,3′-Dideoxyinosin, 2′,3′-Dideoxythioinosin, 2′,3′-Dideoxythioguanosin oder 2′,3′-Dideoxy-2-aminoadenosin ist.

15. Pharmazeutische Zusammensetzung gemäss Anspruch 13, worin der Purinnucleosid-Phosphorylase -Inhibitor entweder (a) Formycin B, oder (b) 8-Aminoguanin, 8-Aminoguanosin, 1-β-D-Ribofuranosyl-1H-1,2,4-triazol-3-carboxamidin, 8-Amino-9-(1,3-dihydroxy-2-propoxymethyl)guanin, 9-Deazaguanosin, 9-Deazainosin, 5′-Deoxy-5′-chlor-9-deazainosin, 5′-Deoxy-5′-iod-9-deazainosin oder 8-Amino-9-deaza-9-(3-thienylmethyl)guanin oder (c) 8-Amino-9-benzylguanin, 8-Amino-9-(2-thienylmethyl)guanin oder 8-Amino-9-(3-thienylmethyl)guanin ist.

16. Pharmazeutische Zusammensetzung gemäss Anspruch 13, worin das 2′,3′-Dideoxypurinnucleosid 2′,3′-Dideoxyadenosin, 2′,3′-Dideoxyguanosin, 2′,3′-Dideoxyinosin, 2′,3′-Dideoxythioinosin, 2′,3′-Dideoxythioguanosin oder 2′,3′-Dideoxy-2-aminoadenosin und der Purinnucleosid-Phosphorylase-Inhibitor entweder

(a) Formycin B oder (b) 8-Aminoguanin, 8-Aminoguanosin, 1-β-D-Ribofuranosyl-1H-1,2,4-triazol-3-carboxamidin, 8-Amino-9-(1,3-dihydroxy-2-propoxymethyl)guanin, 9-Deazaguanosin, 9-Deazainosin, 5′-Deoxy-5′-chlor-9-deazainosin, 5′-Deoxy-5′-iod-9-deazainosin oder 8-Amino-9-deaza-9-(3-thienylmethyl)guanin oder (c) 8-Amino-9-benzylguanin, 8-Amino-9-(2-thienylmethyl)guanin oder 8-Amino-9-(3-thienylmethyl)guanin ist.

17. Pharmazeutische Zusammensetzung gemäss Anspruch 13, worin das 2′,3′-Dideoxypurinnucleosid 2′,3′-Dideoxyinosin ist.

18. Verwendung einer antiretroviral wirksamen Menge eines 2′,3′-Dideoxypurinnucleosids und einer die Purinnucleosid-Phosphorylase inhibierenden Menge eines Purinnucleosid-Phosphorylase -Inhibitors zur Herstellung von pharmazeutischen Präparaten zur Anwendung als Mittel gegen Retrovirusinfektionen, insbesondere gegen HIV-Infektionen.

## Claims

1. A mixture of substances comprising an anti-retrovirally effective amount of a 2′,3′-dideoxypurine nucleoside and a purine nucleoside phosphorylase-inhibiting amount of a purine nucleoside phosphorylase inhibitor.

2. A mixture of substances according to claim 1, wherein the 2′,3′-dideoxypurine nucleoside is a compound of the general formula I

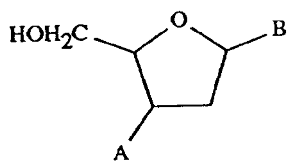

(I)

wherein A is a hydrogen atom, an azide group or a halogen atom; B is the radical of a purine base bound through the 9-position thereof; and the hydroxy group is free or is derivatised as a metabolizable ester, or a pharmaceutically acceptable salt thereof.

3. A mixture of substances according to claim 2, wherein the compound of the general formula I is either 2′,3′-dideoxythioguanosine or 2′,3′-dideoxy-2-aminoadenosine.

4. A mixture of substances according to claim 2, wherein the compound of the general formula I is 2′,3′-dideoxyadenosine, 2′,3′-dideoxyguanosine, 2′,3′-dideoxyinosine or 2′,3′-dideoxythioinosine.

5. A mixture of substances according to claim 2, wherein the compound of the general formula I is 2′,3′-dideoxyadenosine.

6. A mixture of substances according to claim 2, wherein the compound of the general formula I is 2′,3′-dideoxyinosine.

7. A mixture of substances according to any one of claims 1 to 6, wherein the purine nucleoside phosphorylase inhibitor is (a) formycin B, (b) 8-aminoguanine, 8-aminoguanosine, 1-β-D-ribofuranosyl-1H-1,2,4-triazole-3-carboxamidine, 8-amino-9-(1,3-dihydroxy-2-propoxymethyl)guanine, 9-deazaguanosine, 9-deazainosine, 5′-deoxy-5′-chloro-9-deazainosine, 5′-deoxy-5′-iodo-9-deazainosine or 8-amino-9-deaza-9-(3-thienylmethyl)guanine or (c) 8-amino -9-benzylguanine, 8-amino-9-(2-thienylmethyl)guanine or 8-amino-9-(3-thienylmethyl)guanine.

8. A mixture of substances according to claim 7, wherein the purine nucleoside phosphorylase inhibitor is either a compound from group (b) or a compound from group (c).

9. A mixture of substances according to claim 7, wherein the purine nucleoside phosphorylase inhibitor is a compound from group (c).

10. A mixture of substances according to claim 1, wherein the anti-retrovirally effective amount of the 2′,3′-dideoxypurine nucleoside is 0.01 to 20 mg and the purine nucleoside phosphorylase-inhibiting amount of the purine nucleoside inhibitor is 0.5 to 150 mg.

11. A mixture of substances comprising an anti-retrovirally effective amount of a 2′,3′-dideoxypurine nucleoside and a purine nucleoside phosphorylase-inhibiting amount of a purine nucleoside phosphorylase inhibitor for the therapeutic treatment of the human or animal body.

12. A product comprising an anti-retrovirally effective amount of a 2′,3′-dideoxypurine nucleoside and a purine nucleoside phosphorylase-inhibiting amount of a purine nucleoside phosphorylase inhibitor in the presence or absence of one or more pharmaceutically acceptable carriers, in the form of a combination preparation for administration simultaneously or at different times within a period that is short enough to ensure that the purine nucleoside phosphorylase inhibitor prevents, or at least reduces the rate of, the degradation of the 2′,3′-dideoxypurine nucleoside by native purine nucleoside phosphorylase, for the treatment of a condition that responds to treatment with a 2′,3′-dideoxypurine nucleotide.

13. A pharmaceutical composition for the treatment of a condition that responds to treatment with a 2′,3′-dideoxypurine nucleoside, comprising an effective amount of a 2′,3′-dideoxypurine nucleoside, a purine nucleoside phosphorylase-inhibiting amount of a purine nucleoside phosphorylase inhibitor and one or more pharmaceutically acceptable carriers.

14. A pharmaceutical composition according to claim 13, wherein the 2′,3′-dideoxypurine nucleoside is 2′,3′-dideoxyadenosine, 2′,3′-dideoxyguanosine, 2′,3′-dideoxyinosine, 2′,3′-dideoxythioinosine, 2′,3′-dideoxythioguanosine or 2′,3′-dideoxy-2-aminoadenosine.

15. A pharmaceutical composition according to claim 13, wherein the purine nucleoside phosphorylase inhibitor is (a) formycin B, (b) 8-aminoguanine, 8-aminoguanosine, 1-β-D-ribofuranosyl-1H-1,2,4-triazole-3-carboxamidine, 8-amino -9-(1,3-dihydroxy-2-propoxymethyl)guanine, 9-deazaguanosine, 9-deazainosine, 5′-deoxy-5′-chloro-9-deazainosine, 5′-deoxy-5′-iodo-9-deazainosine or 8-amino-9-deaza-9-(3-thienylmethyl)guanine or (c) 8-amino-9-benzylguanine, 8-amino-9-(2-thienylmethyl)guanine or 8-amino -9-(3-thienylmethyl)guanine.

16. A pharmaceutical composition according to claim 13, wherein the 2′,3′-dideoxypurine nucleoside is 2′,3′-dideoxyadenosine, 2′,3′-dideoxyguanosine, 2′,3′-dideoxyinosine, 2′,3′-dideoxythioinosine, 2′,3′-dideoxythioguanosine or 2′,3′-dideoxy-2-aminoadenosine and the purine nucleoside phosphorylase inhibitor is (a) formycin B, (b) 8-aminoguanine, 8-aminoguanosine, 1-β-D-ribofuranosyl -1H-1,2,4-triazole-3-carboxamidine, 8-amino-9-(1,3-dihydroxy-2-propoxymethyl)guanine, 9-deazaguanosine, 9-deazainosine, 5′-deoxy-5′-chloro-9-deazainosine, 5′-deoxy-5′-iodo-9-deazainosine or 8-amino-9-deaza-9-(3-thienylmethyl )guanine or (c) 8-amino-9-benzylguanine, 8-amino -9-(2-thienylmethyl)guanine or 8-amino-9-(3-thienylmethyl)guanine.

17. A pharmaceutical composition according to claim 13, wherein the 2′,3′-dideoxypurine nucleoside is 2′,3′-dideoxyinosine.

18. The use of an anti-retrovirally effective amount of a 2′,3′-dideoxypurine nucleoside and of a purine nucleoside phosphorylase-inhibiting amount of a purine nucleoside-phosphorylase inhibitor for the preparation of a pharmaceutical composition for use against retrovirus infections, especially against HIV infections.

## Revendications

1. Mélange de substances consistant en une quantité antirétrovirale efficace d'un 2′,3′-didésoxypurine-nucléoside et un inhibiteur de la purine-nucléoside-phosphorylase en quantité suffisante pour inhiber cette dernière.

2. Mélange de substances selon revendication 1, dans lequel le 2′,3′-didésoxypurine-nucléoside est un composé de formule générale I

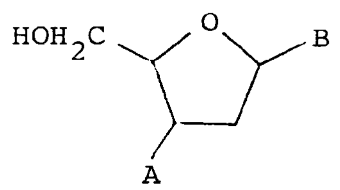

(I)

dans laquelle A représente un atome d'hydrogène, un groupe azide ou un atome d'halogène, B est le radical d'une base purique relié par sa position 9, et le groupe hydroxy est à l'état libre ou à l'état d'ester métabolisable, ou un sel acceptable pour l'usage pharmaceutique d'un tel composé.

3. Mélange de substances selon revendication 2, dans lequel le composé de formule générale I est la 2′,3′-didésoxythioguanosine ou la 2′,3′-didésoxy-2-aminoadénosine.

4. Mélange de substances selon revendication 2, dans lequel le composé de formule générale I est la 2′,3′-didésoxyadénosine, la 2′,3′-didésoxyguanosine, la 2′,3′-didésoxyinosine ou la 2′,3′-didésoxythioinosine.

5. Mélange de substances selon revendication 2, dans lequel le composé de formule générale I est la 2′,3′-didésoxyadénosine.

6. Mélange de substances selon revendication 2, dans lequel le composé de formule générale I est la 2′,3′-didésoxyinosine.

7. Mélange de substances selon une des revendications 1 à 6, dans lequel l'inhibiteur de la purinenucléoside-phosphorylase est soit (a) la formycine B, soit (b) la 8-aminoguanine, la 8-aminoguanosine, la 1-bêta-D-ribofurannosyl-1H-1,2,4-triazole-3-carboxamidine, la 8-amino-9-(1,3-dihydroxy-2-propoxyméthyl)-guanine, la 9-déazaguanosine, la 9-déazasinine, la 5′-désoxy-5′-chloro-9-déazainosine, la 5′-désoxy-5′-iodo-9-déazainosine ou la 8-amino-9-déaza-9-(3-thiénylméthyl)-guanine, soit (c) la 8-amino-9-benzylguanine, la 8-amino-9-(2-thiénylméthyl)-guanine ou la 8-amino-9-(3-thiénylméthyl)-guanine.

8. Mélange de substances selon revendication 7, dans lequel l'inhibiteur de la purine-nucléoside-phosphorylase est un composé du groupe (b) ou du groupe (c).

9. Mélange de substances selon revendication 7, dans lequel l'inhibiteur de la purine-nucléoside-phosphorylase est un composé du groupe (c).

10. Mélange de substances selon revendication 1, dans lequel la quantité antirétrovirale efficace du 2′,3′-didésoxypurine-nucléoside est de 0,01 à 20 mg et la quantité d'inhibiteur de la purine-nucléoside-phosphorylase suffisante pour inhiber cette dernière est de 0,5 à 150 mg.

11. Mélange de substances consistant en une quantité antirétrovirale efficace d'un 2′,3′-didésoxypurine-nucléoside et un inhibiteur de la purine-nucléosidephosphorylase en quantité suffisante pour inhiber cette dernière, pour le traitement thérapeutique de l'organisme humain ou animal.

12. Produit contenant une quantité antirétrovirale efficace d'un 2′,3′-didésoxypurine-nucléoside et un inhiteur de la purine-nucléoside-phosphorylase en quantité suffisante pour inhiber cette dernière, en présence ou en l'absence d'un ou plusieurs véhicules acceptables pour l'usage pharmaceutique, en tant que préparation combinée pour l'administration simultanée ou à des moments différents à l'intérieur d'une période suffisamment courte pour assurer que l'inhibiteur de la purinenucléoside-phosphorylase évite la dégradation du 2′,3′didésoxypurine-nucléoside sous l'action de la purinenucléoside-phosphorylase naturelle, ou au moins pour en diminuer la vitesse, pour le traitement d'un état demandant un traitement par un 2′,3′-didésoxypurine-nucléoside.

13. Composition pharmaceutique pour le traitement d'un état demandant un traitement par du 2′,3′-didésoxypurine-nucléoside, consistant en une quantité efficace d'un 2′,3′-didésoxypurine-nucléoside, un inhibiteur de la purine-nucléoside-phosphorylase en quantité suffisante pour inhiber cette dernière et un ou plusieurs véhicules acceptables pour l'usage pharmaceutique.

14. Composition pharmaceutique selon revendication 13, dans laquelle le 2′,3′-didésoxypurine-nucléoside

est la 2′,3′-didésoxyadénosine, la 2′,3′-didésoxyguanosine, la 2′,3′-didésoxyinosine, la 2′,3′-didésoxythioinosine, la 2′,3′-didésoxythioguanosine ou la 2′,3′-didésoxy-2-aminoadénosine.

**15.** Composition pharmaceutique selon revendication 13, dans laquelle l'inhibiteur de la purine-nucléoside-phosphorylase est soit (a) la formycine B, soit (b) la 8-aminoguanine, la 8-aminoguanosine, la 1-bêta-D-ribofurannosyl-1H-1-2,4-triazole-3-carboxamidine, la 8-amino -9-(1,3-dihydroxy-2-propoxyméthyl)-guanine, la 9-déazaguanosine, la 9-déazainosine, la 5′-désoxy-5′-chloro-9-déazainosine, la 5′-désoxy-5′-iodo-9-déazainosine ou la 8-amino-9-déaza-9-(3-thiénylméthyl)-guanine, soit (c) la 8-amino-9-benzylguanine, la 8-amino-9-(2-thiénylméthyl)-guanine ou la 8-amino-9-(3-thiénylméthyl)-guanine.

**16.** Composition pharmaceutique selon revendication 13, dans laquelle le 2′,3′-didésoxypurine-nucléoside est la 2′,3′-didésoxyadénosine, la 2′,3′-didésoxyguanosine, la 2′,3′- didésoxyinosine, la 2′,3′-didésoxythioinosine, la 2′,3′-didésoxythioguanosine ou la 2′,3′-didésoxy-2-aminoadénosine et l'inhibiteur de la purinenucléoside-phosphorylase est soit (a) la formycine B, soit (b) la 8-aminoguanine, la 8-aminoguanosine, la 1-bêta-D-ribofurannosyl-1H-1,2,4-triazole-3-carboxamidine, la 8-amino-9-(1,3-dihydroxy-2-propoxyméthyl)-guanine, la 9-déazaguanosine, la 9-déazainosine, la 5′-désoxy-5′-chloro-9-déazainosine, la 5′-désoxy-5′-iodo-9-déazainosine ou la 8-amino-9-déaza-9-(3-thiénylméthyl)-guanine, soit (c) la 8-amino-9-benzylguanine, la 8-amino-9-(2-thiénylméthyl)-guanine ou la 8-amino-9-(3-thiénylméthyl)-guanine.

**17.** Composition pharmaceutique selon revendication 13, dans laquelle le 2′,3′-didésoxypurine-nucléside est la 2′,3′-didésoxyinosine.

**18.** Utilisation d'une quantité antirétrovirale efficace d'un 2′,3′-didésoxypurine-nucléoside et d'un inhibiteur de la purine-nucléoside-phosphorylase en quantité suffisante pour inhiber cette dernière, pour la préparation de compositions pharmaceutiques destinées à être utilisées en tant que médicaments pour les infections par rétrovirus, en particulier les infections par virus HIV.